# EUROPÄISCHE PATENTSCHRIFT

(11) **EP 2 394 021 B1**
(45) Veröffentlichungstag und Bekanntmachung des Hinweises auf die Patenterteilung: **28.05.2014**
(21) Anmeldenummer: 10704888.6
(22) Anmeldetag: 22.01.2010
(51) Int. Cl.: E21B 43/16, E21B 41/00

(54) **VERFAHREN ZUR ERDGASFÖRDERUNG AUS KOHLENWASSERSTOFF-HYDRATEN BEI GLEICHZEITIGER SPEICHERUNG VON KOHLENDIOXID IN GEOLOGISCHEN FORMATIONEN**
METHOD FOR PRODUCING NATURAL GAS FROM HYDROCARBON HYDRATES WHILE SIMULTANEOUSLY STORING CARBON DIOXIDE IN GEOLOGICAL FORMATIONS
PROCÉDÉ D'EXTRACTION DE GAZ NATUREL ISSU D'HYDRATES D'HYDROCARBURES AVEC STOCKAGE SIMULTANÉ DE DIOXYDE DE CARBONE DANS DES FORMATIONS GÉOLOGIQUES

(30) Priorität: 04.02.2009 DE 102009007453
(43) Veröffentlichungstag der Anmeldung: 14.12.2011
(73) Patentinhaber: Leibniz-Institut für Meereswissenschaften, 24148 Kiel (DE)
(72) Erfinder: WALLMANN, Klaus, 24114 Kiel (DE); HAECKEL, Matthias, 24116 Kiel (DE)
(74) Vertreter: Appelt, Christian W.
(86) Internationale Anmeldenummer: PCT/DE2010/000059
(87) Internationale Veröffentlichungsnummer: WO 2010/088874

(56) Entgegenhaltungen:
- GB-A- 2 445 120
- JP-A- 2004 003 326

## Beschreibung

Die Erfindung betrifft ein Verfahren zur Förderung von in Gashydraten eingelagertem Methan bei gleichzeitiger Speicherung von Kohlendioxid (CO₂) im geologischen Untergrund.

Gewaltige Mengen an Erdgas sind als festes eisähnliches Methanhydrat im Meeresboden gespeichert. Diese natürlichen Vorkommen enthalten wahrscheinlich mehr Energie und Kohlenstoff (ca. 3000 Gt C) als alle konventionellen Lagerstätten von Kohle, Öl und Gas auf unserem Planeten. Die Gashydrate spielen daher eine bedeutende Rolle als Erdgasquelle der Zukunft. Methanhydrate wurden an fast allen Kontinenträndem ab Wassertiefen von ca. 400 m nachgewiesen. Sie sind nur bei hohen Drücken und niedrigen Temperaturen stabil. Sie treten dort auf, wo genügend organischer Kohlenstoff ins Sediment eingebettet wurde und die Druck- und Temperaturbedingungen die Fixierung von Methan in Methanhydraten erlauben. Viele Küstenanrainerstaaten verfügen über große nationale Vorkommen (z. B. China, Indien, Japan, Südkorea, Brasilien, Chile, USA, Kanada, Norwegen, Russland). Darüber hinaus wurden Methanhydrate an Land unterhalb von mächtigen Permafrost-Ablagerungen nachgewiesen. Diese Hydratvorkommen sind vor allem aus Sibirien, Kanada und Alaska bekannt.

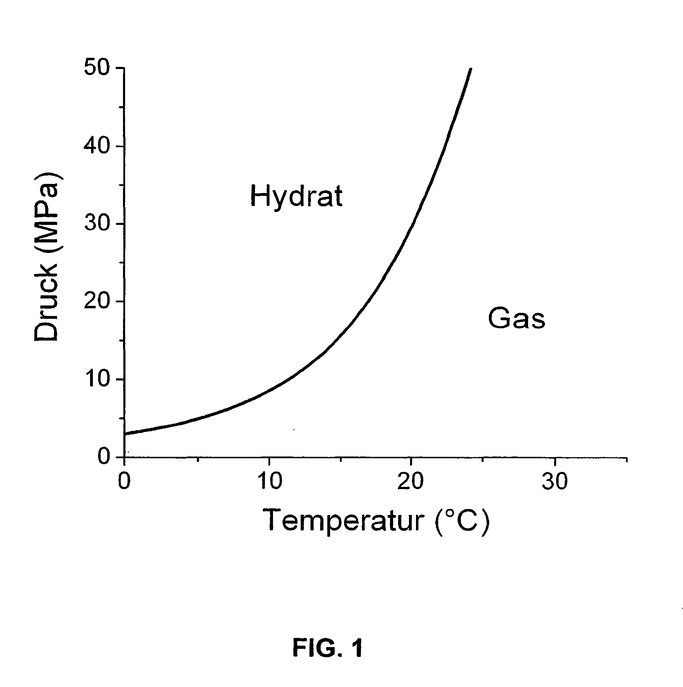

Fig. 1 zeigt das Phasendiagram von Methan im Meerwasser. Die Methanhydrate sind nur bei hohen Drücken und niedrigen Temperaturen stabil. Die Phasengrenze zwischen Hydrat und Gas gilt für reines Methanhydrat vom Strukturtyp I und für Meerwasser mit einem Salzgehalt von 35 Gew.-%.

Die Phasengrenzen gelten für reines Methanhydrat vom Strukturtyp I. Methanhydrate kommen in unterschiedlichen Strukturtypen vor. Der Typ I ist dabei die häufigste und am weitesten verbreitete Variante.

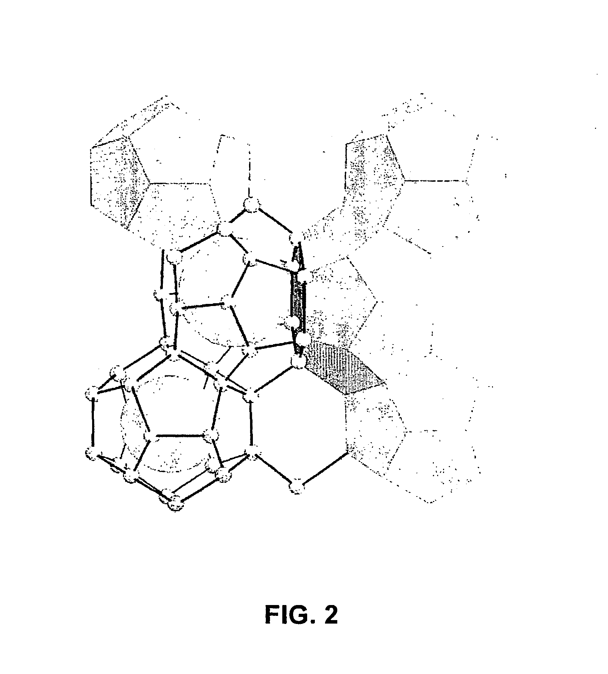

In Fig. 2 ist ein Methanhydrat-Cluster des Strukturtyps I gezeigt, bei diesem Typ kommen auf ein Methan-Molekül im Mittel 5,7 Wassermoleküle. Die Methanmoleküle sind durch die großen Kugeln dargestellt, während die durch schwarze Striche verbundenen kleinen Kugeln das durch Wassermoleküle aufgebaute Hydratgitter darstellen.

Methanhydrat-Lagerstätten werden zurzeit weltweit erschlossen, um Erdgas zu fördern. Zur Gewinnung von Erdgas müssen die Hydrate zunächst im geologischen Untergrund zersetzt werden. Dabei wird das in den Wasserkäfigen der Hydrate fixierte Methan als Gas freigesetzt, das dann mit konventionellen Techniken über eine oder mehrere Bohrungen gefördert werden kann. Zurzeit werden im Wesentlichen die folgenden unterschiedlichen Ansätze verfolgt:
- der Druck in der Lagerstätte wird erniedrigt;
- die Temperatur in der Lagerstätte wird erhöht;
- chemische Substanzen werden zugesetzt, um die Hydrate zu zersetzen.

Aus der US 7,222,673 ist bekannt, ohne Zerstörung der Hydratstruktur, Methan aus den Gashydraten gegen Kohlendioxid (CO₂) auszutauschen. Dabei werden die Hydrate mit flüssigem CO₂ in Kontakt gebracht. Die Reaktion läuft ohne externe Energiezufuhr ab, da die entstehenden CO₂-Hydrate stabiler sind als die natürlichen Methanhydrate. Diese Art der Erdgasgewinnung bietet den zusätzlichen Vorteil, dass gleichzeitig CO₂, welches als klimarelevantes Treibhausgas für die Erderwärmung mit verantwortlich ist, sicher im Untergrund gespeichert werden kann und somit aus der Atmosphäre entfernt wird. Ein Nachteil dieses Verfahrens ist die geringe Geschwindigkeit der Austauschreaktion unter Erhalt der Hydratstruktur, die nur sehr kleine Förderraten erlaubt.

In der W02005/076904 wird eine Methode beschrieben, CO₂ unter dem Meeresboden zu lagern, in dem gasförmiges CO₂ in Methanhydratfelder eingeleitet wird. Es kommt zur Bildung von CO₂-Hydrat, die dabei freiwerdende Wärme führt zur Dissoziation des Methanhydrates und zur Freisetzung des Methans. Die Sammlung und Nutzung des freigewordenen Methangases ist vorgesehen. Für die Nutzung des freigewordenen Methangases zur Energieerzeugung durch Verbrennung erweist sich der hohe Gehalt an gasförmigem CO₂ als Nachteil. Die möglichen Förderaten sind ebenfalls gering, da die Freisetzung des Methans aus dem Methanhydrat erst mit Hilfe der durch die CO₂-Hydratbildung frei werdende Wärme möglich wird.

Ein weiteres Beispiel ist in dem Dokument GB 2 445 120 A zu finden, welches als nächstliegenden Stand der Technik angesehen wird.

Daher ist es Aufgabe dieser Erfindung eine Methode zu schaffen, Kohlenwasserstoffe, insbesondere Methan, aus den Hydraten mit höheren als den bisher möglichen Förderaten zu gewinnen und dabei gleichzeitig CO₂ in geologischen Formationen zu speichern.

Die Aufgabe wird durch das Verfahren mit den Merkmalen von Anspruch 1 gelöst. Die Unteransprüche geben vorteilhafte Ausführungen der Erfindung wieder.

Es wird vorgeschlagen, zur Gewinnung von Erdgas und zur Speicherung von CO₂ im Untergrund, superkritisches CO₂ in die Hydratvorkommen zu injizieren. Dabei werden die Methanhydrate sehr schnell und in einem großen Bereich um den Injektionsbohrung thermisch und chemisch zersetzt, so dass hohe Erdgasförderraten erreicht werden können.

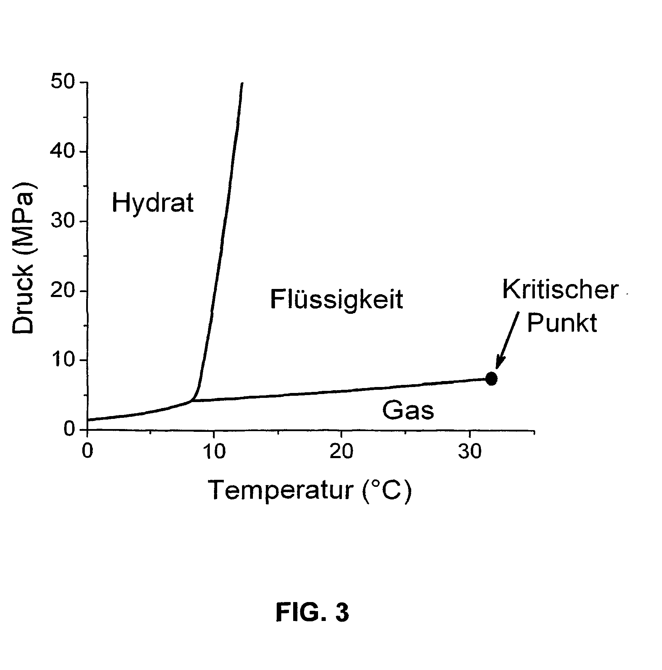

Fig. 3 zeigt das Phasendiagramm von CO₂ in Meerwasser in Abhängigkeit von Druck und Temperatur. Die Phasengrenze zwischen Hydrat und flüssigem CO₂ gilt für reines CO₂-Hydrat vom Strukturtyp I und für Meerwasser mit einem Salzgehalt von 35 Gew.-%. Der kritische Punkt von CO₂ liegt bei 7,4 MPa und 31,48 °C. Bei höheren Temperaturen und Drücken geht CO₂ in die so genannte überkritische bzw. superkritische Phase über. Das besondere an dieser Phase ist, dass es dabei zwischen gasförmigem und flüssigem Zustand keine abrupten Übergänge und Energiebarneren gibt; es verschwindet die Unterscheidbarkeit zwischen der Gas- und der Flüssigkeitsphase. Superkritisches CO₂ ist somit etwas anderes als flüssiges oder gasförmiges CO₂. Es besteht aus CO₂-Clustern, die nur lose miteinander verbunden sind. Es hat sehr spezielle Eigenschaften, die für die Erdgasförderung aus Hydraten besonders günstig sind.

Superkritisches CO₂ reagiert sehr begierig und schnell mit den Methanhydraten, da die Methanhydrate sowohl thermisch als auch chemisch zersetzt werden. Die Methanhydrate sind bei Temperaturen von mehr als 31,48 °C instabil und werden daher durch das superkritische CO₂ aufgeschmolzen. Diese thermische Zersetzung der Methanhydrate verläuft sehr viel schneller als der langsame Austausch von Gasmolekülen bei Erhalt der Hydratstruktur. Gleichzeitig werden die Wasserkäfige durch die chemische Reaktion mit den CO₂-Clustern angegriffen und zersetzt. Aufgrund der gleichzeitig wirkenden thermischen und chemischen Energien verläuft die Freisetzung von Erdgas aus Methanhydrat mit superkritischem CO₂ schneller als mit flüssigem oder gasförmigem CO₂ oder mit Warmwasser gleicher Temperatur.

Das injizierte superkritische CO₂-Fluid hat eine geringe Viskosität und hohe Mobilität. Die Wärme kann sich somit im Untergrund durch die rasche Konvektion des niedrigviskosen. superkritischen CO₂ im Porenraum schnell ausbreiten, so dass die Methanhydrate in einem großen Bereich um die Injektionsbohrung aufgeschmolzen werden. Die erfindungsgemäße Freisetzung von Erdgas aus Methanhydrat verläuft aufgrund der Fließeigenschaften von superkritischem CO₂ deutlich effektiver als bei Einsatz von Warmwasser gleicher Temperatur, da superkritisches CO₂ bei gleicher Temperatur eine weitaus geringere Viskosität und höhere Ausbreitungsgeschwindigkeit hat als Warmwasser.

Ein zusätzlicher Vorteil des vorgeschlagenen Verfahrens liegt darin, dass aufgrund der lokalen Temperaturerhöhung in der Nähe des Injektionsbohrung kein oder nur wenig CO₂-Hydrat entsteht, so dass eine Verstopfung der Zuleitungen und des Porenraums vermieden wird.

Weiterhin werden bei dem vorgeschlagenen Verfahren der Porenraum und die verbleibenden Formationswässer mit CO₂ gesättigt, so dass die Rückreaktion, d. h. die Bildung von Methanhydrat aus dem freigesetzten Erdgas, vermieden wird.

Mit dem vorgeschlagenem Verfahren können wirtschaftlich attraktive Erdgasförderraten erreicht werden. Damit kann auf weitere Methoden zur Zersetzung der Methanhydrate, wie die Injektion von Warmwasser, die Erniedrigung des Druckes oder die Zusetzung von chemischen Substanzen verzichtet werden.

Das superkritische CO₂ verbleibt im Untergrund. Es wird sich dort im Laufe der Zeit langsam abkühlen und schließlich in CO₂-Hydrat verwandeln.

Erfindungsgemäß wird das Methanhydrat erst aufgeschmolzen und zersetzt, das CO₂-Hydrat entsteht zu einem späteren Zeitpunkt, nachdem die Erdgasförderung partiell oder vollständig abgeschlossen ist und die Wärme das Reservoir durch Konduktion verlassen hat.

Das Verfahren kann in verschiedenen Varianten realisiert werden. Es ist z. B. möglich, das superkritische CO₂ über eine getrennte Injektionsbohrung in die Lagerstätte einzubringen. Das Bohrloch muss dazu thermisch isoliert werden, um den Wärmeverlust zwischen Bohrplattform und Lagerstätte zu minimieren. Das freigesetzte Methangas kann über eine separate Bohrung gefördert werden. Es ist auch möglich, die CO₂-Injektion und die Erdgasförderung durch ein und dieselbe Bohrung zu führen. Weiterhin können Horizontalbohrungen durchgeführt werden oder Hydrofracturing-Verfahren eingesetzt werden, um die Durchlässigkeit der Hydrat-führenden Sedimentschichten zu erhöhen.

## Patentansprüche

1. Verfahren zur Gewinnung von Methan aus Methanhydraten, mit den Schritten:
- Zuleiten von Kohlendioxid zu Methanhydratvorkommen,
- Wirkenlassen des Kohlendioxids auf das Methanhydrat unter Freisetzen von Methan und Einlagern des Kohlendioxids als Kohlendioxidhydrat,
- Abführen des freigesetzten Methans,
**dadurch gekennzeichnet, dass**
das zugeleitete Kohlendioxid superkritisches Kohlendioxid ist.

2. Verfahren nach Anspruch 1, **dadurch gekennzeichnet, dass** das zugeleitete superkritische Kohlendioxid unter einem Druck von größer als 7,4 MPa steht und auf eine Temperatur von größer als 31,48 °C temperiert ist.

3. Verfahren nach einem der vorhergehenden Ansprüche, **dadurch gekennzeichnet, dass** das superkritische Kohlendioxid natürlichen Methanhydratlagerstätten zugeführt wird.

4. Verfahren nach Anspruch 3, **dadurch gekennzeichnet, dass** die natürlichen Methanhydratlagerstätten submers liegen.

5. Verfahren nach einem der vorhergehenden Ansprüche, **dadurch gekennzeichnet, dass** das superkritische Kohlendioxid dem Methanhydratvorkommen in thermisch isolierten Leitungen zugeführt wird.

6. Verfahren nach einem der vorhergehenden Ansprüche, **dadurch gekennzeichnet, dass** das superkritische Kohlendioxid in das Methanhydratvorkommen injiziert wird.

## Claims

1. A method for extracting methane from methane hydrates, having the following steps:
- feeding carbon monoxide to methane hydrate deposits,
- letting the carbon dioxide act on the methane hydrate while releasing methane and intercalating the carbon dioxide as carbon dioxide hydrate;
- discharging the released methane,
**characterized in that**
the fed carbon dioxide is super-critical carbon dioxide.

2. The method according to Claim 1, **characterized in that** the fed super-critical carbon dioxide is held at a pressure of more than 7.4 MPa and at a temperature of more than 31.48°C.

3. The method according to one of the preceding claims, **characterized in that** the super-critical carbon dioxide is fed to natural methane hydrate deposits.

4. The method according to Claim 3, **characterized in that** the natural methane hydrate deposits are submersed.

5. The method according to one of the preceding claims, **characterized in that** the super-critical carbon dioxide is fed to methane hydrate deposits in thermally insulated pipes.

6. The method according to one of the preceding claims, **characterized in that** the super-critical carbon dioxide is injected into the methane hydrate deposits.

## Revendications

1. Procédé de récupération de méthane à partir d'hydrates de méthane, comprenant les étapes suivantes:
- Introduire le dioxyde de carbone dans des gisements d'hydrates de méthane,
- laisser agir le dioxyde de carbone sur les hydrates de méthane pour libérer le méthane et stocker le dioxyde de carbone sous forme d'hydrate de dioxyde de carbone,
- extraire le méthane libéré,
**caractérisé en ce que**
le dioxyde de carbone introduit est un dioxyde de carbone supercritique.

2. Procédé selon la revendication 1, **caractérisé en ce que** le dioxyde de carbone supercritique introduit est soumis à une pression supérieure à 7,4 MPa et maintenu à une température supérieure à 31,48°C.

3. Procédé selon l'une des revendications précédentes, **caractérisé en ce que** le dioxyde de carbone supercritique est introduit dans des gisements naturels d'hydrates de méthane.

4. procédé selon la revendication 3, **caractérisé en ce que** les gisements naturels d'hydrates de méthane sont submergés.

5. procédé selon l'une des revendications précédentes, **caractérisé en ce que** le dioxyde de carbone supercritique est introduit dans les sites d'hydrates de méthane à l'aide de canalisations thermiquement isolées.

6. Procédé selon l'une des revendications précédentes, **caractérisé en ce que** le dioxyde de carbone supercritique est injecté dans le site d'hydrates de méthane.
